# EUROPEAN PATENT APPLICATION

(11) **EP 4 796 627 A1**
(43) Date of publication of application: **26.08.2026**
(21) Application number: 24891431.9
(22) Date of filing: 13.11.2024
(51) Int. Cl.: C12M 3/00, C12M 1/00

(54) **CELL CULTURE SUBSTRATE, METHOD FOR PRODUCING SAME, AND CELL CULTURE KIT**

(30) Priority: 15.11.2023 JP 2023194519; 22.12.2023 JP 2023216903
(71) Applicant: Tosoh Corporation, Shunan-shi, Yamaguchi 746-8501 (JP)
(72) Inventor: IMAIZUMI, Yu, Yokkaichi-shi, Mie 510-8540 (JP); KADOTA, Junpei, Yokkaichi-shi, Mie 510-8540 (JP)
(74) Representative: Novagraaf International SA
(86) International application number: PCT/JP2024/040340
(87) International publication number: WO 2025/105395

(57) **Abstract**

The present invention relates to a cell culture substrate comprising: a substrate, and a layer containing a hydrophilic polymer covering at least a part of a surface of the substrate, wherein the cell culture substrate has the following (A) region and a (B) region:
(A) region having cell adhesiveness and cell proliferative properties
(B) region being adjacent to the (A) region and not having cell adhesiveness or cell proliferative properties; and
the (A) region includes a recess formed in the layer containing the hydrophilic polymer, the recess has an inclined surface and a bottom surface, and an inclination angle of the inclined surface is 40° or more and 110° or less.

## Description

### Technical Field

The present invention relates to a cell culture substrate, a method for producing the same, and a cell culture kit.

### Background Art

Pluripotent stem cells such as embryonic stem cells (ES cells) and induced pluripotent stem cells (iPS cells) are cells having the ability to differentiate into various tissues of a living body (pluripotency), and have attracted great attention as a cell source in the field of regenerative medicine and for drug discovery screening. In order to apply pluripotent stem cells to regenerative medicine and drug discovery screening, it is necessary to differentiate pluripotent stem cells into desired cells, and at that time, it is necessary to form cell aggregates of pluripotent stem cells. In addition, although pluripotent stem cells can be differentiated into various cells, it is known that the optimal size of cell aggregates varies depending on the type of cells after differentiation, and it is desirable to control the size and to produce cell aggregates having a uniform size.

As a cell culture substrate for forming a cell aggregate, for example, Patent Literature 1 discloses a cell culture substrate having a substrate and a stimulus-responsive polymer covered on the substrate, wherein the stimulus-responsive polymer is a block copolymer having a water-insoluble block segment and a stimulus-responsive block segment, and the cell culture substrate has (A) an island-like region having cell proliferative properties and stimulation responsiveness and an area of 0.001 to 5 mm², and (B) a region adjacent to the (A) region and having no cell proliferative properties.

### Citation List

### Patent Literature

Patent Literature 1: Japanese Unexamined Patent Publication No. 2020-62009

### Summary of Invention

### Technical Problem

The cell culture substrate disclosed in Patent Literature 1 has a specific (A) region and a specific (B) region, and in the (A) region, spheroids can be efficiently formed, and spheroids having a uniform size and an arbitrary shape can be formed. Meanwhile, in the cell culture substrate disclosed in Patent Literature 1, plasma treatment (Isotropic etching: under a gas pressure of 20 Pa, a conductive current was 20 mA, and an irradiation time was 10 seconds.) is used when the (A) the region is formed. However, the present inventors have found that the cell culture substrate produced by this method has room for improvement in terms of uniformity of the shape of the (A) region and clarity of the uneven shapes of the (A) region and the (B) region. By improving the uniformity of the shape of the (A) region and the clarity of the uneven shapes of the (A) region and the (B) region, a cell aggregate having a uniform size and shape can be more stably obtained.

An object of the present invention is to provide a cell culture substrate capable of stably obtaining a cell aggregate having a uniform size and shape. It is also an object of the present invention to provide a method for producing the cell culture substrate.

### Solution to Problem

The present invention relates to a cell culture substrate comprising: a substrate, and a layer containing a hydrophilic polymer covering at least a part of a surface of the substrate, wherein the cell culture substrate has the following (A) region and (B) region,
(A) region having cell adhesiveness and cell proliferative properties
(B) region being adjacent to the (A) region and not having cell adhesiveness or cell proliferative properties; and
the (A) region includes a recess formed in the layer containing the hydrophilic polymer, the recess has an inclined surface and a bottom surface, and an inclination angle of the inclined surface is 40° or more and 110° or less.

The present invention also relates to a method for producing a cell culture substrate, the method comprising: a covering step of covering at least a part of a surface of a substrate with a composition containing a hydrophilic polymer to form a layer containing the hydrophilic polymer; and a patterning step of subjecting a part of the surface of the layer containing the hydrophilic polymer to plasma treatment to form the (A) region at the plasma-treated part.

The present invention further relates to a cell culture kit including the cell culture substrate according to the present invention provided with a partition member having a plurality of cylindrical partition walls capable of partitioning a surface on a side for culturing cells.

The present invention includes, for example, the following inventions.
[1] A cell culture substrate comprising:
   a substrate, and a layer containing a hydrophilic polymer covering at least a part of a surface of the substrate, wherein the cell culture substrate has the following (A) region and (B) region:
      (A) region having cell adhesiveness and cell proliferative properties
      (B) region being adjacent to the (A) region and not having cell adhesiveness or cell proliferative properties; and
   the (A) region includes a recess formed in the layer containing the hydrophilic polymer, the recess has an inclined surface and a bottom surface, and an inclination angle of the inclined surface is 40° or more and 110° or less.
[2] The cell culture substrate according to [1], wherein a maximum depth of the (A) region is 1 nm or more and 500 nm or less.
[3] The cell culture substrate according to [1] or [2], wherein at least a part of the bottom surface of the (A) region is composed of the substrate.
[4] The cell culture substrate according to any one of [1] to [3], wherein a layer thickness of the layer containing a hydrophilic polymer is 10 nm or more and 500 nm or less.
[5] The cell culture substrate according to any one of [1] to [4], wherein a planar region defined by a boundary between the (A) region and the (B) region is an ellipse having an area of 0.001 mm² or more and 5 mm² or less and an aspect ratio of 1 or more and 1.1 or less.
[6] The cell culture substrate according to any one of [1] to [5], wherein a ratio (diameter/maximum depth) of a diameter (nm) of an opening of the (A) region to a maximum depth (nm) of the (A) region is 500 or more and 3000 or less.
[7] The cell culture substrate according to any one of [1] to [6], wherein a minimum distance between the (A) regions is 400 µm or more and 10000 µm or less.
[8] The cell culture substrate according to any one of [1] to [7], wherein the water contact angle of the (A) region is 20° or more and 110° or less.
[9] The cell culture substrate according to any one of [1] to [8], wherein the (A) region is formed by reactive ion etching treatment.
[10] The cell culture substrate according to any one of [1] to [9], wherein a thickness of the substrate is 0.01 mm or more and 0.5 mm or less.
[11] A method for producing a cell culture substrate according to any one of [1] to [10], comprising: a covering step of covering at least a part of a surface of a substrate with a composition containing a hydrophilic polymer to form a layer containing the hydrophilic polymer; and
   a patterning step of subjecting a part of the surface of the layer containing the hydrophilic polymer to plasma treatment to form the (A) region at the plasma-treated part.
[12] The method for producing a cell culture substrate according to [11], wherein
   the hydrophilic polymer has reactivity to an active energy ray, and
   the covering step includes irradiating a layer containing the hydrophilic polymer with an active energy ray to immobilize the layer on a surface of the substrate.
[13] The method for producing a cell culture substrate according to [11] or [12], wherein the plasma treatment is reactive ion etching treatment.
[14] The production method according to any one of [11] to [13], further comprising, after the patterning step, a bonding step of bonding a plate having a through-hole having a cross-sectional area in an in-plane direction of 0.05 cm² or more and 100 cm² or less to the substrate on a surface side of the substrate covered with the layer containing a hydrophilic polymer.
[15] The production method according to any one of [11] to [14], further comprising, after the patterning step, a temperature-responsive layer forming step of forming a layer containing a temperature-responsive polymer by covering a surface of the layer containing a hydrophilic polymer subjected to a plasma treatment with a composition containing the temperature-responsive polymer.
[16] A cell culture kit comprising a cell culture substrate, wherein
   the cell culture substrate has a partition member having a plurality of cylindrical partition walls capable of partitioning a surface on a side for culturing cells provided on the cell culture substrate according to any one of [1] to [10].
[17] The cell culture substrate according to any one of [1] to [10], wherein a carbon ratio of carboxyl groups to total carbon in the (A) region (R_{COOH}) is 0.25% or more.

### Advantageous Effects of Invention

According to the present invention, it is possible to provide a cell culture substrate capable of stably obtaining a cell aggregate having a uniform size and shape. According to the present invention, it is also possible to provide a method for producing the cell culture substrate.

### Brief Description of Drawings

FIG. 1 is a schematic view (cross-sectional view) of a cell culture substrate according to an embodiment.
FIG. 2 is a schematic view for explaining a method of calculating an inclination angle.
FIG. 3 is a schematic view (perspective view) of a substrate in which a layer containing a hydrophilic polymer is formed on a surface after a covering step in the production method according to an embodiment.
FIG. 4 is a schematic view (perspective view) of a cell culture substrate after a patterning step in a production method according to an embodiment.
FIG. 5 is a schematic view (perspective view) of a cell culture substrate after a bonding step in the production method according to an embodiment.

### Description of Embodiments

Hereinafter, embodiments for carrying out the present invention will be described in detail. Note that the present invention is not limited to the following embodiments, and the following embodiments can be modified and implemented within a range in which the above-described effects can be obtained.

As used herein, the term "cell aggregate" means a three-dimensional aggregate of cells formed by aggregation of a plurality of cells. The shape of the three-dimensional aggregate may be an ellipsoid shape such as a spherical shape, or may be a shape such as a hemispherical shape. These shapes may be shapes with gaps formed by folding of sheet-like cells, or may be hollow shapes. One example of a cell aggregate is a spheroid.

In the present specification, the term "temperature-responsiveness" indicates that the degree of hydrophilicity/hydrophobicity changes depending on a temperature change. Further, the boundary temperature at which the degree of hydrophilicity/hydrophobicity changes is referred to as a "response temperature".

In the present specification, the term "biologically derived substance" means a substance present in the body of an organism and a substance equivalent to the substance and chemically synthesized. The substance present in the body of an organism may be a natural product or may be artificially synthesized by a genetic recombination technology or the like. The biologically derived substance is not particularly limited, and examples thereof include nucleic acids, proteins, and polysaccharides, which are basic materials constituting a living body; and nucleotides, nucleosides, amino acids, various sugars, lipids, vitamins, and hormones which are components thereof.

In the present specification, the term "cell adhesiveness" indicates ease of adhesion to a substrate or a cell culture substrate at a culture temperature, and the phrase "having cell adhesiveness" indicates that cells can adhere to the substrate or the cell culture substrate directly or via a biologically derived substance at the culture temperature. In addition, the phrase "having no cell adhesiveness" indicates that cells cannot adhere to a substrate or a cell culture substrate directly or via a biologically derived substance at a culture temperature. The method of evaluating the presence or absence of cell adhesiveness is not particularly limited, and examples thereof include a method of adding the above-described biologically derived substance subjected to a labeling treatment such as a fluorescent label to the region and confirming the presence or absence of adsorption of the substance, and a method of measuring a water contact angle of the region.

In the present specification, the term "cell proliferative properties" refers to the ease of cell proliferation at a culture temperature, and "having cell proliferative properties" refers to the ability of cells to proliferate at a culture temperature. In addition, the phrase "having no cell proliferative properties" indicates that cells cannot proliferate at a culture temperature. The term "High cell proliferative properties" indicates that the cells proliferate into more cells when compared in the same culture period.

The cell culture substrate according to the present embodiment includes a substrate and a layer containing a hydrophilic polymer that covers at least a part of a surface of the substrate, and has the following (A) region and (B) region.
(A) Island-like region with cell adhesiveness and cell proliferative properties
(B) Region being adjacent to the (A) region and having no cell adhesiveness or cell proliferative properties

Here, the (A) region includes a recess formed in the layer containing a hydrophilic polymer, the recess has an inclined surface and a bottom surface, and an inclination angle of the inclined surface is 40° or more and 110° or less.

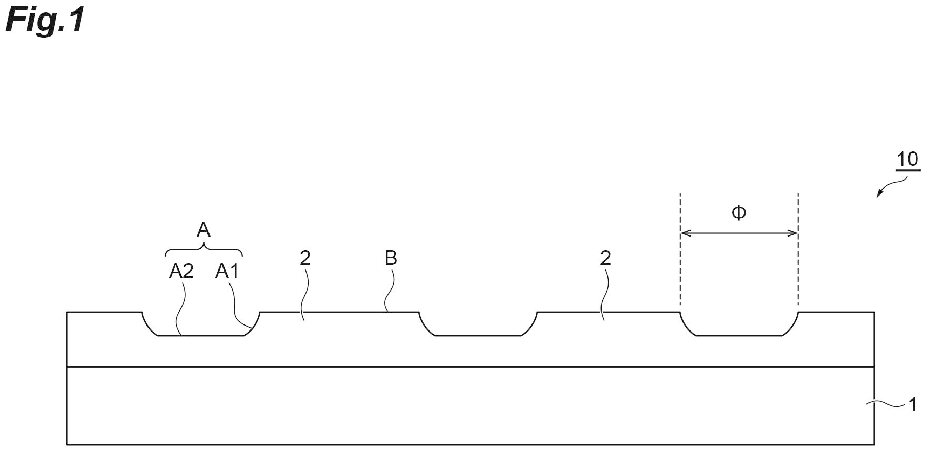

FIG. 1 is a schematic view (cross-sectional view) of a cell culture substrate according to an embodiment. A cell culture substrate 10 shown in FIG. 1 includes a substrate 1 and a layer 2 covering the surface of the substrate 1 and containing a hydrophilic polymer. In FIG. 1, A and B indicate a (A) region and a (B) region, respectively. In FIG. 1, A1 and A2 indicate an inclined surface and a bottom surface of the recess, respectively, and A1 and A2 together constitute the (A) region. The layer thickness of the layer 2 containing a hydrophilic polymer means a distance from a surface where the substrate 1 and the layer 2 containing a hydrophilic polymer are in contact with each other to a surface of the (B) region (region indicated by B in FIG. 1). In the cell culture substrate 10 shown in FIG. 1, the bottom surface A2 of the (A) region is the layer 2 containing a hydrophilic polymer, but it is not limited thereto, and for example, at least a part or all of the bottom surface A2 of the (A) region may be the substrate 1. Similarly, at least a part of the inclined surface A1 of the (A) region may be the substrate 1.

The maximum depth of the (A) region means the distance in the out-of-plane direction between the bottom surface of the (A) region and the surface of the (B) region. The maximum depth of the (A) region may be, for example, 1 nm or more and 2000 nm or less, and may be 1 nm or more and 500 nm or less. When the maximum depth of the (A) region is 500 nm or less, the number of dead cells captured by unevenness can be further suppressed, and the number of dead cells mixed in the cell aggregate can be further reduced. This makes it possible to further increase the cell viability of the cell aggregates. When the maximum depth of the (A) region is 500 nm or less, adhesion of air bubbles to the uneven portion is further suppressed. When the adhesion of the air bubbles is suppressed, it is not necessary to perform degassing to remove the air bubbles or to repeatedly discharge and suck the culture medium using a pipettor, and the operability is further improved. When the maximum depth of the (A) region is 1 nm or more, living cells that spontaneously move (migrate) on the cell culture substrate more easily gather in the (A) region, so that the cell viability of cell aggregates can be further enhanced. The maximum depth of the (A) region is more preferably 400 nm or less, still more preferably 350 nm or less since it is suitable for further increasing the cell viability of the formed cell aggregates.

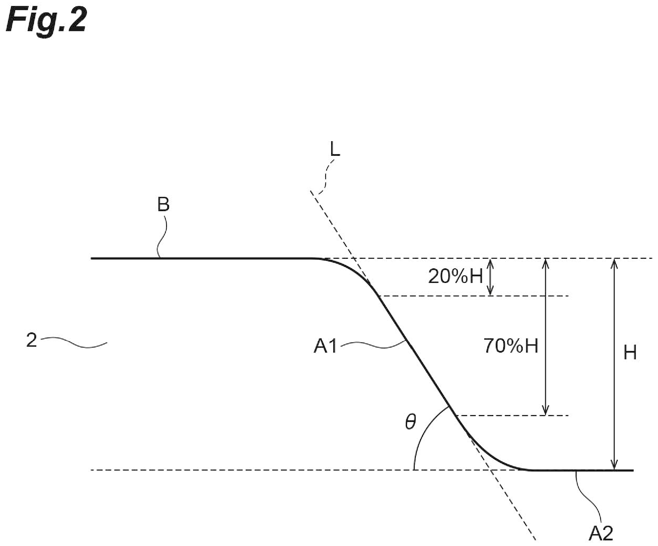

In the present specification, the term "inclination angle of the inclined surface" means an angle formed between an approximate curve obtained by linearly approximating the inclined surface in a range of 20% or more and 70% or less of the maximum depth of the (A) region and the bottom surface of the (A) region on a plane perpendicularly intersecting the substrate along an axis passing through the center (center of gravity) of the bottom surface of the (A) region. FIG. 2 is a schematic view (cross-sectional view) for explaining a method of calculating an inclination angle. In FIG. 2, H represents the maximum depth (that is, the distance in the out-of-plane direction between the bottom surface A2 of the (A) region and the surface of the (B) region) of the (A) region. The shape of the (A) region in a plane that perpendicularly intersects the substrate along an axis passing through the center (center of gravity ) of the bottom surface of the (A) region can be measured, for example, using a stylus profiling system (manufactured by Bruker Corporation, trade name: DEKTAK XT). Based on the maximum depth H of the (A) region, the inclined surface A1 is linearly approximated within a range of 20% (20%×H) or more and 70% (70%×H) or less of the maximum depth H. The linear approximation can be performed, for example, by a least squares method. Next, an angle θ formed by the obtained approximate straight line L and the bottom surface A2 of the (A) region is calculated. This angle θ is the inclination angle of the inclined surface. As illustrated in FIG. 2, when the recess is tapered as the shape of the (A) region approaches the bottom surface A2, the angle θ is more than 0° and 90° or less. In addition, in a case where the recess expands in a reverse tapered shape as the shape of the (A) region approaches the bottom surface A2, the angle θ is 90° or more and less than 180°.

In the cell culture substrate according to the present embodiment, the inclination angle of the inclined surface is 40° or more and 110° or less. When the inclination angle of the inclined surface is within this range, the uneven shape becomes clear, so that a cell aggregate having a uniform size and shape can be more stably obtained. From the same viewpoint, the inclination angle of the inclined surface is preferably 50° or more, more preferably 60° or more, still more preferably 65° or more, even more preferably 70° or more, still more preferably 75° or more, and particularly preferably 80° or more. From the same viewpoint, the inclination angle of the inclined surface is preferably 100° or less, more preferably 95° or less, still more preferably 90° or less, and even more preferably less than 90°. The inclination angle of the inclined surface may be 40° or more and 100° or less, 40° or more and 95° or less, 40° or more and 90° or less, 40° or more and less than 90°, 50° or more and 110° or less, 50° or more and 100° or less, 50° or more and 95° or less, 50° or more and 90° or less, 50° or more and less than 90°, 60° or more and 110° or less, 60° or more and 100° or less, 60° or more and 95° or less, 60° or more and 90° or less, 60° or more and less than 90°, 65° or more and 100° or less, 65° or more and 95° or less, 65° or more and 90° or less, 65° or more and less than 90°, 70° or more and 110° or less, 70° or more and 100° or less, 70° or more and 95° or less, 70° or more and 90° or less, 70° or more and less than 90°, 75° or more and 110° or less, 75° or more and 100° or less, 75° or more and 95° or less, 75° or more and 90° or less, 75° or more and less than 90°, 80° or more and 110° or less, 80° or more and 100° or less, 80° or more and 95° or less, 80° or more and 90° or less, or 80° or more and less than 90°. Here, in a case where the (A) region is formed by etching such as plasma treatment to be described later, as treatment conditions such as an increase in treatment time are enhanced, the (A) region becomes deeper and the inclination angle tends to increase. When the maximum depth of the (A) region is 1 nm or more and 500 nm or less and the inclination angle of the inclined surface is less than 90°, the mass productivity of the cell culture substrate is excellent, and a cell aggregate having a uniform size and shape can be stably obtained using the cell culture substrate.

The substrate used for the cell culture substrate according to the present embodiment is not particularly limited, but is preferably formed of at least one selected from the group consisting of polystyrene, polyethylene, polyethylene terephthalate, polycarbonate, a cycloolefin polymer, cellulose acetate, nitrocellulose, and polyvinylidene fluoride, more preferably formed of at least one selected from the group consisting of polystyrene, polyethylene terephthalate, polycarbonate, and a cycloolefin polymer, further preferably formed of at least one selected from polystyrene, polyethylene terephthalate, and polycarbonate, and most preferably formed of polystyrene or polycarbonate.

Since it is suitable for observing the cells cultured on the cell culture substrate with a high magnification phase contrast microscope, the refractive index of the substrate measured with a D line (wavelength 589 nm) is preferably 1.4 or more and 1.6 or less, more preferably 1.45 or more and 1.6 or less, and particularly preferably 1.5 or more and 1.55 or less. When the refractive index of the substrate is in these ranges, spherical aberration in observation with a phase contrast microscope of cells can be reduced, and a clear phase difference image can be obtained. For reducing spherical aberration, the thickness of the substrate is preferably 0.5 mm or less, more preferably 0.4 mm or less, particularly preferably 0.3 mm or less, most preferably 0.2 mm or less. On the other hand, the thickness of the substrate is preferably 0.01 mm or more, more preferably 0.05 mm or more, particularly preferably 0.1 mm or more, and most preferably 0.15 mm or more since it is suitable for suppressing out-of-focus of the entire observation range due to deflection of the substrate at the time of microscopic observation.

The thickness of the substrate is preferably 0.01 mm or more and 0.5 mm or less, more preferably 0.05 mm or more and 0.4 mm or less, further preferably 0.1 mm or more and 0.3 mm or less, and particularly preferably 0.15 mm or more and 0.2 mm or less. When the thickness of the substrate is in this range, the phase difference image of the cell becomes clearer.

Since it is suitable for observing cells cultured on a cell culture substrate with a high magnification fluorescence microscope, the fluorescence intensity (autofluorescence intensity) of the substrate at excitation wavelengths of 350 nm, 488 nm, and 647 nm (when irradiated with excitation light having these wavelengths, respectively) is preferably smaller than the fluorescence intensity (autofluorescence intensity) of a 1.2 mm-thick polystyrene plate excited by light having the same excitation wavelength, more preferably 80% or less of the fluorescence intensity of a 1.2 mm-thick polystyrene plate, particularly preferably 50% or less of the fluorescence intensity of a 1.2 mm-thick polystyrene plate, and most preferably 10% or less of the fluorescence intensity of a 1.2 mm-thick polystyrene plate. Fluorescent dyes excited at excitation wavelengths of 350 nm, 488 nm, and 647 nm are frequently used in fluorescence observation of cells. When the autofluorescence intensity of the substrate at these wavelengths is equal to or less than a certain value, a clear fluorescence image of the cell can be obtained.

The shape of the substrate is not particularly limited, and may be a planar shape such as a plate or a film, or may be a shape such as a fiber, a porous particle, a porous membrane, or a hollow fiber. The shape of the substrate may be a shape of a container (cell culture dishes such as Petri dishes, flasks, plates, bags, etc.) generally used for cell culture or the like. From the viewpoint of ease of culture operation, the shape of the substrate is preferably a planar shape such as a plate or a film, or a shape of a flat porous membrane.

The layer thickness of the layer containing the hydrophilic polymer may be, for example, 10 nm or more and 500 nm or less. When the layer thickness of the layer containing the hydrophilic polymer is 10 nm or more and 500 nm or less, it is possible to allow cells to adhere and proliferate only in the (A) region, and it is easy to collect cells in the (A) region by migration of cells, and it is possible to increase the cell viability of cell aggregates. Here, the term "layer thickness" of the layer containing the hydrophilic polymer refers to a length in an out-of-plane direction from an interface between the substrate and the layer containing the hydrophilic polymer to an interface (excluding the (A) region) on a side opposite to the substrate of the layer containing the hydrophilic polymer. When the layer thickness exceeds 10 nm, the thickness can be calculated by measuring a cross-sectional image with a transmission electron microscope using an ultrathin section of a cell culture substrate prepared with a microtome, measuring the distances of 10 randomly selected points, and averaging the measured distances. When the layer thickness is 10 nm or less, the thickness can be measured using an ellipsometer. The layer thickness is more preferably 10 nm or more, still more preferably 30 nm or more, 40 nm or more, or 50 nm or more since it is suitable for suppressing adhesion of cells to the (B) region. In addition, the layer thickness is more preferably 200 nm or less, still more preferably 180 nm or less, 160 nm or less, 150 nm or less, 140 nm or less, or 120 nm or less, since it is suitable for enhancing the cell viability of cell aggregates by collecting cells in the (A) region by migration of cells. The layer thickness of the layer containing the hydrophilic polymer may be, for example, 10 nm or more and 200 nm or less, 10 nm or more and 180 nm or less, 10 nm or more and 160 nm or less, 10 nm or more and 150 nm or less, 10 nm or more and 140 nm or less, 10 nm or more and 120 nm or less, 30 nm or more and 200 nm or less, 30 nm or more and 180 nm or less, 30 nm or more and 160 nm or less, 30 nm or more and 150 nm or less, 30 nm or more and 140 nm or less, 30 nm or more and 120 nm or less, 40 nm or more and 200 nm or less, 40 nm or more and 180 nm or less, 40 nm or more and 160 nm or less, 40 nm or more and 150 nm or less, 40 nm or more and 140 nm or less, 40 nm or more and 120 nm or less, 50 nm or more and 200 nm or less, 50 nm or more and 180 nm or less, 50 nm or more and 160 nm or less, 50 nm or more and 150 nm or less, 50 nm or more and 140 nm or less, or 50 nm or more and 120 nm or less.

The hydrophilic polymer contains a phosphorylcholine group or a hydroxyl group. When the hydrophilic polymer contains a phosphorylcholine group or a hydroxyl group, a region covered with the hydrophilic polymer can be a region to which cells do not adhere. In addition, since such a hydrophilic polymer does not need to be completely decomposed and removed, the region can be made into a region having cell adhesiveness and cell proliferative properties by performing plasma treatment, and a decomposition product of the hydrophilic polymer is less likely to be mixed into cells. Other than containing a phosphorylcholine group or a hydroxyl group, the type of the hydrophilic polymer is not particularly limited, and examples of the commercially available product include Lipidure(R)CM5206 (manufactured by NOF CORPORATION), Lipidure(R)CM2001 (manufactured by NOF CORPORATION), and BIOSURFINE(R)-AWP (manufactured by Toyo Gosei Co., Ltd.). As a commercially available substrate covered with a hydrophilic polymer, PrimeSurface (R) (manufactured by Sumitomo Bakelite Co., Ltd.), EZ-BindShut (R) (manufactured by AGC Technoglass Co., Ltd.), EZ-BindShutII (R) (manufactured by AGC Technoglass Co., Ltd.), and the like can be suitably used.

The hydrophilic polymer preferably contains a compound represented by the following general formula (1), a compound represented by the following general formula (2), or a compound represented by the following general formula (3). 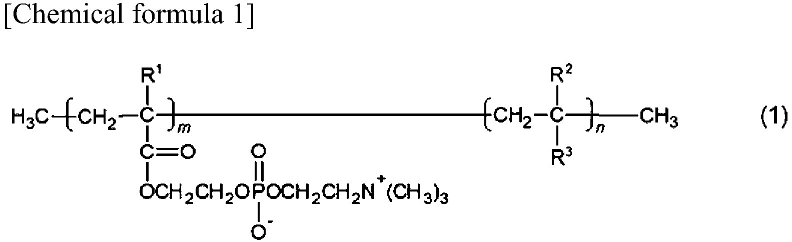 [In the general formula (1), R¹ and R² each independently represent a hydrogen atom or a methyl group, R³ represents a hydrogen atom or an arbitrary organic group, and m and n each independently represent a positive integer.] 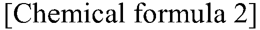 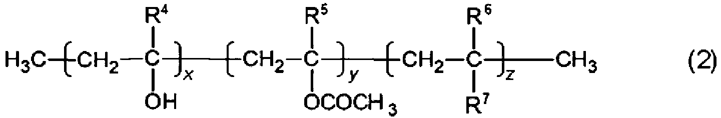 [In the general formula (2), R⁴, R⁵ and R⁶ each independently represent a hydrogen atom or a methyl group, R⁷ represents a hydrogen atom or an arbitrary organic group, and x, y and z each independently represent a positive integer.] 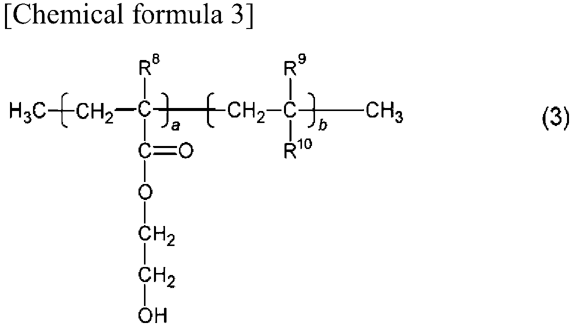 [In the general formula (3), R⁸ and R⁹ each independently represent a hydrogen atom or a methyl group, R¹⁰ represents a hydrogen atom or an arbitrary organic group, and a and b each independently represent a positive integer.]

When the hydrophilic polymer includes the compound represented by the general formula (1), the compound represented by the general formula (2), or the compound represented by the general formula (3), cells easily adhere and proliferate, and it is suitable for forming a cell aggregate having a uniform shape in the (A) region. In the general formula (1), the general formula (2), or the general formula (3), R³, R⁷, and R¹⁰ are each preferably a hydrophobic group or a functional group having reactivity to an active energy ray (for example, UV, electron beam, and the like) since they are suitable for immobilizing a hydrophilic polymer on a substrate. As the hydrophobic group, a linear or cyclic alkyl group such as a methyl group, an ethyl group, a propyl group, a butyl group, or a cyclohexyl group can be suitably used. Examples of the functional group having reactivity to an active energy ray (for example, UV, electron beam, and the like) include an azide group, an acrylate group, a methacrylate group, a vinyl group, and an epoxy group, and an azide group can be suitably used.

The (A) region consists of a recess formed in the layer containing the hydrophilic polymer. Here, the recess has an inclined surface and a bottom surface. In the cell culture substrate according to the present embodiment, the inclination angle of the inclined surface in the (A) region is 40° or more and 110° or less. In the (A) region, both the inclined surface and the bottom surface may be in the layer containing the hydrophilic polymer, and a part of the inclined surface and/or a part or all of the bottom surface may be in the substrate.

The (A) region is grasped as an island-like region when the cell culture substrate is observed from the layer side containing the hydrophilic polymer in a direction orthogonal to the substrate. At this time, the outer periphery of the island-like region corresponds to the boundary between the (A) region and the (B) region. In the present specification, a planar region defined by a boundary (that is, the outer periphery of the island-like region) between the (A) region and the (B) region may be referred to as an opening. In FIG. 1, φ represents the diameter of the opening (in the present specification, also referred to as a spot diameter). Note that being an island-like region means that the region exists independently of a region other than the region. When the (A) region is the island-like region as described above, living cells are concentrated in the (A) region as compared with a case where the region is not the island-like region as described above (for example, in the case of a stripe structure or the like), so that the cell aggregate can be more efficiently produced.

The shape of the opening (the planar region defined by the boundary between the (A) region and the (B) region) is not particularly limited, and can be appropriately set according to the shape of the intended cell aggregate, and examples thereof include an ellipse (including a circle), a polygon, and a closed shape formed by a straight line and a curved line. In addition, since it is suitable for producing a cell aggregate having a shape close to a sphere, the shape of the opening is preferably an ellipse (including a circle) or a polygon, more preferably an ellipse (including a circle) or a rectangle, still more preferably an ellipse (including a circle) or a square, and most preferably an ellipse (including a circle).

The aspect ratio of the shape of the opening (the planar region defined by the boundary between the (A) region and the (B) region) is preferably 1 or more and 2 or less, more preferably 1 or more and 1.5 or less, still more preferably 1 or more and 1.1 or less, and most preferably 1 or more and 1.05 or less since it is suitable for producing a cell aggregate having a shape close to a sphere. Here, the term "aspect ratio" indicates a ratio of a maximum diameter (major axis) to a minimum diameter (minor axis) of the shape, i.e., major axis/minor axis.

The standard deviation of the aspect ratio/average aspect ratio of the shape of the opening (the planar region defined by the boundary between the (A) region and the (B) region) is preferably 80% or less, more preferably 50% or less, still more preferably 20% or less, and most preferably 5% or less since it is suitable for producing a cell aggregate having a uniform size and shape.

The area of the opening (the planar region defined by the boundary between the (A) region and the (B) region) may be, for example, 0.001 mm² or more and 5 mm² or less. The area is preferably 0.005 mm² or more and 1 mm² or less, more preferably 0.01 mm² or more and 0.5 mm² or less, still more preferably 0.015 mm² or more and 0.25 mm² or less, and most preferably 0.02 mm² or more and 0.2 mm² or less.

The standard deviation/average area of the area of the opening (the planar region defined by the boundary between the (A) region and the (B) region) is preferably 80% or less, more preferably 50% or less, still more preferably 20% or less, and most preferably 5% or less since it is suitable for producing a cell aggregate having a uniform size and shape.

The shape of the bottom surface of the (A) region may be the same as or different from the shape of the opening. The shape of the bottom surface of the (A) region is not particularly limited, and can be appropriately set according to the shape of a target cell aggregate, and examples thereof include an ellipse (including a circle), a polygon, and a closed shape formed by a straight line and a curved line. In addition, since it is suitable for producing a cell aggregate having a shape close to a sphere, the shape of the opening is preferably an ellipse (including a circle) or a polygon, more preferably an ellipse (including a circle) or a rectangle, still more preferably an ellipse (including a circle) or a square, and most preferably an ellipse (including a circle).

The aspect ratio of the shape of the bottom surface of the (A) region may be the same as or different from the aspect ratio of the shape of the opening. Since it is suitable for producing a cell aggregate having a shape close to a sphere, the aspect ratio of the shape of the bottom surface of the (A) region is preferably 1 or more and 2 or less, more preferably 1 or more and 1.5 or less, still more preferably 1 or more and 1.1 or less, and most preferably 1 or more and 1.05 or less.

Since it is suitable for producing a cell aggregate having a uniform size and shape, the standard deviation/average aspect ratio of the aspect ratio of the shape of the bottom surface of the (A) region is preferably 80% or less, more preferably 50% or less, still more preferably 20% or less, and most preferably 5% or less.

The area of the bottom surface of the (A) region may be the same as or different from the area of the opening. The area of the bottom surface of the (A) region may be, for example, 0.001 mm² or more and 6 mm² or less. The area is preferably 0.001 mm² or more and 5 mm² or less, more preferably 0.005 mm² or more and 1 mm² or less, still more preferably 0.01 mm² or more and 0.5 mm² or less, even more preferably 0.015 mm² or more and 0.25 mm² or less, and most preferably 0.02 mm² or more and 0.2 mm² or less.

Since it is suitable for producing a cell aggregate having a uniform size and shape, the standard deviation/average area of the area of the bottom surface of the (A) region is preferably 80% or less, more preferably 50% or less, still more preferably 20% or less, and most preferably 5% or less.

In the (A) region, the ratio (diameter/maximum depth) of the diameter (nm) of the opening of the (A) region to the maximum depth (nm) of the (A) region can be, for example, 100 or more and 20000 or less. The diameter/maximum depth is preferably 500 or more and 3000 or less. As a result, the uneven shape becomes clear, and a cell aggregate having a uniform size and shape can be more stably obtained. The diameter/maximum depth is more preferably 500 or more and 2000 or less, still more preferably 500 or more and 800 or less. The "diameter of the opening" means an average of a major axis diameter (maximum diameter) and a minor axis diameter (minimum diameter) of the opening. The diameter of the opening is indicated by φ in FIG. 1.

In addition, the minimum distance between the (A) regions is preferably 400 µm or more and 10000 µm or less, more preferably 500 µm or more and 8000 µm or less, still more preferably 1000 µm or more and 5000 µm or less, and most preferably 2000 µm or more and 4000 µm or less since it is suitable for increasing the oxygen concentration around cells and increasing the survival rate of cell aggregates.

The (A) region can be formed, for example, by forming a layer containing a hydrophilic polymer on the surface of the substrate and then modifying a part of the surface of the layer containing a hydrophilic polymer by plasma treatment. Since the (A) region is a region in which the surface of the layer containing the hydrophilic polymer is modified by plasma treatment or the like, the (A) region becomes a region having cell adhesiveness and cell proliferative properties. Since the (A) region can be patterned by short-time plasma treatment or the like, mass productivity of the cell culture substrate can be enhanced.

The plasma treatment described above can also be performed in the presence of an introduction gas such as oxygen, hydrogen, nitrogen, ammonia, or argon. By using the introduction gas described above, a hydrophilic functional group is generated in the material, and a region having cell adhesiveness and cell proliferative properties can be formed. Examples of the type of the functional group include a hydroxyl group, a carbonyl group, an aldehyde group, a carboxyl group, an ether bond, an ester bond, an amino group, and a nitro group depending on the introduction gas used for the plasma treatment. The use of oxygen is preferable since various functional groups of the above-described group, such as a hydroxyl group, a carbonyl group, an aldehyde group, a carboxyl group, an ether bond, an ester bond, and a nitro group, are generated. In particular, when a carboxyl group, which is a highly polar functional group, is generated, the hydrophilicity of the material is greatly modified, and cell adhesiveness and cell proliferative properties are also improved, which is preferable.

When the material of the surface of the cell culture substrate is an organic substance and the functional group to be generated is a carboxyl group, the carbon ratio (R_{COOH}) of the carboxyl group to the total carbon in the (A) region is preferably 0.25% or more, more preferably 0.3% or more, 0.5% or more, or 0.8% or more, most preferably 1.2% or more. The carbon ratio (R_{COOH}) of the carboxyl group to the total carbon in the (A) region may be, for example, 10% or less, 8% or less, 6% or less, 5% or less, 4% or less, or 2% or less. The carbon ratio (R_{COOH}) of the carboxyl group to the total carbon in the (A) region may be, for example, 0.25% or more and 10% or less, 0.25% or more and 8% or less, 0.25% or more and 6% or less, 0.25% or more and 5% or less, 0.25% or more and 4% or less, 0.25% or more and 2% or less, 0.3% or more and 10% or less, 0.3% or more and 8% or less, 0.3% or more and 6% or less, 0.3% or more and 5% or less, 0.3% or more and 4% or less, 0.3% or more and 2% or less, 0.5% or more and 10% or less, 0.5% or more and 8% or less, 0.5% or more and 6% or less, 0.5% or more and 5% or less, 0.5% or more and 4% or less, 0.5% or more and 2% or less, 0.8% or more and 10% or less, 0.8% or more and 8% or less, 0.8% or more and 6% or less, 0.8% or more and 5% or less, 0.8% or more and 4% or less, 0.8% or more and 2% or less, 1.2% or more and 10% or less, 1.2% or more and 8% or less, 1.2% or more and 6% or less, 1.2% or more and 5% or less, 1.2% or more and 4% or less, or 1.2% or more and 2% or less. When the carbon ratio of carboxyl groups to total carbon (R_{COOH}) in the (A) region falls within the above-mentioned range, it becomes possible to provide a cell culture substrate having the cell adhesiveness and the proliferative property shown in the present application. In addition, R_{COOH} is preferably 0.7% or more and 1.0% or less from the viewpoint of reducing the enlargement rate.

In the present specification, the carbon ratio of carboxyl groups to the total carbon in the (A) region (R_{COOH}) is determined by performing gas phase chemical modification using trifluoroethanol, di-t-butylcarbodiimide, and pyridine, measuring the amount of surface functional groups in the (A) region by X-ray photoelectron spectroscopy under the following conditions, and then calculating the ratio from the following formula derived from the reaction formula of the gas phase chemical modification. 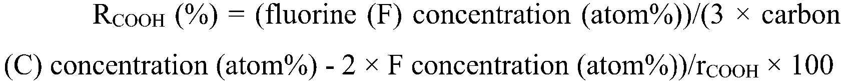 RCOOH (%) = (fluorine (F) concentration (atom%))/(3 × carbon (C) concentration (atom%) - 2 × F concentration (atom%))/rCOOH × 100 r_{COOH}: Reaction rate

### <Measurement conditions>

X-ray: monochrome Al-Kα ray (output: 25 W)
Energy resolution: Wide scan spectrum 117.40 eV
High resolution spectrum 93.90 eV
Charge correction: C1s main peak (284.8 eV)

In addition, the ratio of the peak intensity of 287 eV/the peak intensity of 285 eV in the C1s spectrum when XPS (X-ray photoelectron spectroscopy) for the (A) region is measured is preferably 0.05 or more larger than the ratio of the peak intensity of 287 eV/the peak intensity of 285 eV in the C1s spectrum of XPS measurement for the (B) region. In this case, since the difference in cell proliferative properties between the (A) region and the (B) region can be increased, it is possible to grow many cells in the (A) region, and uniform cell aggregates are easily formed in the (A) region. Since it is more suitable for forming uniform cell aggregates, the ratio of the peak intensity of 287 eV/the peak intensity of 285 eV in the C1s spectrum of XPS measurement for the (A) region is more preferably larger than the ratio of the peak intensity of 287 eV/the peak intensity of 285 eV in the C1s spectrum of XPS measurement for the (B) region by 0.07 or more, still more preferably by 0.1 or more, and most preferably by 0.15 or more.

The method for adjusting the ratio of the peak intensity of 287 eV/the peak intensity of 285 eV in the C1s spectrum in XPS measurement to the above-described range is not particularly limited, but a method is preferable in which only a part of the layer containing a hydrophilic polymer formed on the surface of the substrate (a portion scheduled to become the (A) region) is subjected to plasma treatment. Examples of the method for subjecting only a portion to be the (A) region to plasma treatment include a method in which a layer containing a hydrophilic polymer formed on a surface of a substrate is covered with a mask prepared by laser processing, and plasma treatment is performed from above the mask. Conditions such as the plasma intensity and the plasma irradiation time can be appropriately adjusted so that the difference between the ratio of the peak intensity of 287 eV/the peak intensity of 285 eV in the C1s spectrum of the XPS measurement for the (A) region and the ratio of the peak intensity of 287 eV/the peak intensity of 285 eV in the C1s spectrum of the XPS measurement for the (B) region falls within the above-described range. However, in the case of plasma treatment, by using a gas containing oxygen and nitrogen as an introduction gas, the difference tends to fall within the above-described range.

The plasma irradiation time is preferably 5 seconds or more and 10 minutes or less, more preferably 10 seconds or more and 5 minutes or less, and most preferably 30 seconds or more and 3 minutes or less. As the introduction gas in the plasma treatment, air, nitrogen, or oxygen is preferably used, and oxygen is more preferably used. The gas pressure of the introduction gas is preferably 1 Pascal (Pa) or more and less than 20 Pascal, more preferably 1 Pascal or more and 15 Pascal or less, still more preferably 1 Pascal or more and 10 Pascal or less. By not excessively increasing the gas pressure, it is easier to set the inclination angle of the inclined surface in the (A) region within the above-described preferable range. Furthermore, since it becomes easier to set the inclination angle of the inclined surface in the (A) region within the above-described preferable range, the plasma treatment is particularly preferably performed by reactive ion etching treatment (RIE). RIE is also referred to as anisotropic etching.

The (A) region preferably has a water contact angle of 20° or more and 110° or less. The wettability of the (A) region can be enhanced by modifying a part of the surface of the layer containing the hydrophilic polymer by the plasma treatment described above. The water contact angle of the (A) region is more preferably 40° or more and 80° or less, still more preferably 50° or more and 70° or less, and most preferably 60° or more and 70° or less. When the water contact angle of the (A) region is within the above range, the wettability of the (A) region is further enhanced, and cell adhesiveness and cell proliferative properties are further improved. In addition, the water contact angle of the (A) region is preferably 20° or more and 40° or less, and more preferably 25° or more and 45° or less from the viewpoint of reducing the enlargement rate. The water contact angle of the (A) region can be measured, for example, using the following equation, where θ_{A} is an angle formed between a liquid surface and the surface of the (A) region when a water droplet is dropped on the surface of the (A) region and the droplet is stationary. ${θ}_{A} = 2 arctan \left(h/r\right)$

(In the above formula, θ_{A} represents a water contact angle, h represents a height of a droplet, and r represents a radius of the droplet.)

Since the (A) region is suitable for peeling off the cultured cell aggregate, the region may have temperature-responsiveness. When the (A) region has temperature-responsiveness, the response temperature is preferably 50°C or lower, more preferably 35°C or lower since cells can be cultured at a temperature close to body temperature when culturing the cells on the cell culture substrate. In addition, the response temperature is particularly preferably 25°C or lower since it is suitable for suppressing cell peeling-off when an operation such as medium replacement is performed during culture. Furthermore, since it is possible to form cell aggregates by cooling operation at a temperature that does not damage the cells, the response temperature is preferably 4°C or higher, more preferably 10°C or higher, and still more preferably 15°C or higher.

When the (A) region has temperature-responsiveness, for example, a layer containing a temperature-responsive polymer having a layer thickness of 1 nm or more and 100 nm or less may be further provided on the surface of the layer containing a hydrophilic polymer (the surface including the (A) region and the (B) region). By having a layer containing a temperature-responsive polymer having a layer thickness of 1 nm or more and 100 nm or less, it is possible to impart temperature-responsiveness to the (A) region without impairing the respective characteristics of the (A) region and the (B) region formed on the surface of the layer containing a hydrophilic polymer. The layer thickness of the layer containing the temperature-responsive polymer is more preferably 3 nm or more and 50 nm or less, still more preferably 5 nm or more and 40 nm or less, and most preferably 10 nm or more and 35 nm or less, since it is suitable for imparting temperature-responsiveness to the (A) region without impairing the characteristics of each of the (A) region and the (B) region. The suitable layer thickness of the temperature-responsive polymer, which allows cells to be peeled off and recovered by temperature responsiveness after culture without impairing cell proliferative properties, varies depending on the cells to be cultured, and can be appropriately adjusted within the range of the layer thickness exemplified above.

The temperature-responsive polymer is preferably a block copolymer having a water-insoluble block segment and a temperature-responsive block segment. When the temperature-responsive polymer is such a block copolymer, mass productivity of the cell culture substrate can be enhanced, and contamination of the produced cell aggregate with the temperature-responsive polymer can be suppressed. The constituent unit ratio of the temperature-responsive block segment contained in the temperature-responsive polymer is preferably 70wt% or more, more preferably 80wt% or more, particularly preferably 90wt% or more, most preferably 92wt% or more since it is suitable for rapidly peeling off cell aggregates from the cell culture substrate.

In addition, since the temperature-responsive polymer is a block copolymer having a water-insoluble block segment and a temperature-responsive block segment, temperature-responsiveness can be imparted to the surface of the cell culture substrate by a simple method in which a solution containing a temperature-responsive polymer is dropped on the surface of the cell culture substrate and dried. In addition, since the layer formed at this time has the above-mentioned preferable layer thickness of the temperature-responsive polymer, even if the entire surface of the layer containing the hydrophilic polymer is covered with the temperature-responsive polymer, the respective characteristics of the (A) region and the (B) region are less impaired.

Examples of the monomer unit constituting the temperature-responsive block segment include (meth)acrylamide compounds such as acrylamide and methacrylamide; N-alkyl-substituted (meth)acrylamide derivatives such as N,N-diethylacrylamide, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-t-butylacrylamide, N-ethoxyethylacrylamide, N-ethoxyethylmethacrylamide, N-tetrahydrofurfurylacrylamide, and N-tetrahydrofurfurylmethacrylamide; N,N-dialkyl-substituted (meth)acrylamide derivatives such as N,N-dimethyl (meth)acrylamide, N,N-ethylmethylacrylamide, and N,N-diethylacrylamide; (meth)acrylamide derivatives having a cyclic group, such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine; vinyl ethers such as methyl vinyl ether; and proline derivatives such as N-proline methyl ester acrylamide. Since these are suitable for setting the response temperature to 0 to 50°C, N,N-diethylacrylamide, N-n-propylacrylamide, N-isopropylacrylamide, N-n-propylmethacrylamide, N-ethoxyethylacrylamide, N-tetrahydrofurfurylacrylamide, and N-tetrahydrofurfurylmethacrylamide are preferable, N-n-propylacrylamide and N-isopropylacrylamide are more preferable, and N-isopropylacrylamide is particularly preferable. In addition, when a medium at room temperature is used for medium replacement in culture operation, N-n-propylacrylamide and N-proline methyl ester acrylamide are preferable since they are suitable for setting the response temperature of the block copolymer to a temperature lower than room temperature.

Examples of the monomer unit constituting the water-insoluble block segment include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, and n-tetradecyl methacrylate. In addition, since it is suitable for firmly immobilizing the block copolymer on the substrate, those having a reactive group are preferable, and examples thereof include 4-azidophenyl acrylate, 4-azidophenyl methacrylate, 2-((4-azidobenzoyl)oxy)ethyl acrylate, and 2-((4-azidobenzoyl)oxy)ethyl methacrylate. Furthermore, from the viewpoint of being suitable for enhancing cell proliferative properties, a structure having an aromatic ring is preferable, and examples thereof include 2-hydroxyphenyl acrylate, 2-hydroxyphenyl methacrylate, 3-hydroxyphenyl acrylate, 3-hydroxyphenyl methacrylate, 4-hydroxyphenyl acrylate, 4-hydroxyphenyl methacrylate, N-(2-hydroxyphenyl)acrylamide, N-(2-hydroxyphenyl)methacrylamide, N-(3-hydroxyphenyl)acrylamide, N-(3-hydroxyphenyl)methacrylamide, N-(4-hydroxyphenyl)acrylamide, N-(4-hydroxyphenyl)methacrylamide, and styrene.

The water-insoluble block segment may also include a repeating unit that controls the response temperature of the block copolymer. Examples of the repeating unit for controlling the response temperature of the block copolymer include, but are not particularly limited to, hydrophilic or hydrophobic components, such as those having an amino group such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl]acrylamide; those having a betaine group such as N-(3-sulfopropyl)-N-methacryloyloxyethyl-N,N-dimethylammonium betaine and N-methacryloyloxyethyl-N,N-dimethylammonium-α-N-methylcarboxybetaine; those having a polyethylene glycol group or a methoxyethyl group such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those having an acrylate group such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those having a phosphorylcholine group such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxydecyl phosphorylcholine, ω-(meth)acryloyl(poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and ω-(meth)acrylamide(poly)oxyethylene phosphorylcholine.

The (B) region is adjacent to the (A) region and has no cell adhesiveness or cell proliferative properties. When the (B) region is a region adjacent to the (A) region and having no cell proliferative properties, cell aggregates are formed only in the (A) region when cells are cultured, and it is possible to form a state in which no cell is present around a part or all of the (A) region. In addition, since it is suitable for uniformizing the size and shape of the cell aggregate to be produced, it is preferable that the (B) region has neither cell proliferative properties nor cell adhesiveness.

The shape of the (B) region is not limited except for being adjacent to the (A) region, but since it is suitable for producing a cell aggregate having a uniform size and shape, the (B) region is preferably adjacent to a length of 20% or more of the boundary line of the (A) region, more preferably 50% or more, still more preferably 80% or more, and it is most preferable that the entire periphery of the (A) region is the (B) region.

The area ratio of the (A) region to the (B) region is not particularly limited, but the area of the (A) region is preferably 10% or more, more preferably 30% or more, still more preferably 50% or more, and most preferably 70% or more with respect to the total area of the (A) region and the (B) region, since it is suitable for increasing the number of cell aggregates that can be produced per unit area of the cell culture substrate. In addition, the area of the (B) region is preferably 20% or more, more preferably 40% or more, still more preferably 60% or more, and most preferably 80% or more with respect to the total area of the (A) region and the (B) region since it is suitable for providing a sufficient distance between the plurality of (A) regions and suppressing the cell aggregates of the plurality of (A) regions from fusing into a non-uniform shape.

The cell culture substrate may include a layer containing a biologically derived substance on the surface as necessary. The layer containing the biologically derived substance may be present on the entire surface of the cell culture substrate or may be present only on the surface of the (A) region. The biologically derived substance is not particularly limited, and examples thereof include Matrigel, laminin, fibronectin, vitronectin, and collagen.

These biologically derived substances may be natural products, products artificially synthesized by a genetic recombination technology or the like, fragments cleaved by a restriction enzyme or the like, synthetic proteins or synthetic peptides obtained by chemically synthesizing substances equivalent to these biologically derived substances, or the like.

As Matrigel, commercially available products such as Matrigel (manufactured by Corning Incorporated) and Geltrex (manufactured by Thermo Fisher Scientific) can be suitably used from the viewpoint of easy availability.

The type of laminin is not particularly limited, but for example, Laminin 511, Laminin 521, Laminin-511-E8 fragment, and the like, which are reported to exhibit high activity against α6β1 integrin expressed on the surface of human iPS cells, can be used. Laminin may be a natural product, may be artificially synthesized by a genetic recombination technology or the like, or may be a synthetic protein or a synthetic peptide obtained by chemically synthesizing a substance equivalent to laminin. Commercially available products such as iMatrix-511 (manufactured by NIPPI Corporation) and the like can be suitably used from the viewpoint of easy availability.

Vitronectin may be a natural product, may be artificially synthesized by a genetic recombination technology or the like, or may be a synthetic protein or a synthetic peptide obtained by chemically synthesizing a substance equivalent to vitronectin. From the viewpoint of easy availability, commercially available products such as vitronectin, human plasma derived (manufactured by Wako Pure Chemical Industries, Ltd.), synthemax (manufactured by Corning Incorporated), and Vitronectin (VTN-N) (manufactured by Thermo Fisher Scientific) can be suitably used.

Fibronectin may be a natural product, may be artificially synthesized by a genetic recombination technology or the like, or may be a synthetic protein or a synthetic peptide obtained by chemically synthesizing a substance equivalent to fibronectin. From the viewpoint of easy availability, commercially available products such as a fibronectin solution, human plasma-derived (manufactured by Wako Pure Chemical Industries, Ltd.), and Retronectin (manufactured by Takara Bio Inc.) can be suitably used.

The type of collagen is not particularly limited, but for example, typeI collagen, typeIV collagen, or the like can be used. Collagen may be a natural product, may be artificially synthesized by a genetic recombination technology or the like, or may be a synthetic peptide obtained by chemically synthesizing a substance equivalent to collagen. From the viewpoint of easy availability, commercially available products such as collagen I, human (manufactured by Corning Incorporated), collagen IV, human (manufactured by Corning Incorporated), and the like can be suitably used.

It is preferable that the biologically derived substance is immobilized on the cell culture substrate by a non-covalent bond from the viewpoint of being capable of suppressing denaturation of the biologically derived substance and enhancing cell proliferative properties. Here, the term "non-covalent bond" refers to a bonding force other than covalent bonds derived from intermolecular forces such as electrostatic interactions, water-insoluble interactions, hydrogen bonds, π-π interactions, dipole-dipole interactions, London dispersion forces, and other van der Waals interactions. Immobilization of the biologically derived substance on the block copolymer may be performed by a single bonding force or a combination of two or more.

The method for immobilizing the biologically derived substance is not particularly limited, but for example, a method for immobilizing the biologically derived substance by applying a solution of the biologically derived substance to the cell culture substrate for a predetermined time, or a method for adsorbing and immobilizing the biologically derived substance on the cell culture substrate by adding the biologically derived substance to a culture solution when culturing cells can be suitably used.

The cell culture substrate according to the present embodiment may be provided with a structure for dividing each cell aggregate by, for example, providing a partition plate (for example, a plate having a through hole having a cross-sectional area in an in-plane direction of 0.05 cm² or more and 100 cm² or less) on the substrate as necessary.

The cell culture substrate according to the present embodiment may be sterilized. The sterilization method is not particularly limited, but high-pressure steam sterilization, UV sterilization, γ-ray sterilization, ethylene oxide gas sterilization, and the like can be used. From the viewpoint of suppressing denaturation of the block copolymer, high-pressure steam sterilization, UV sterilization, and ethylene oxide gas sterilization are preferable. From the viewpoint of suppressing deformation of the substrate, UV sterilization or ethylene oxide gas sterilization is more preferable. From the viewpoint of excellent mass productivity, ethylene oxide gas sterilization is preferable.

The cell to be cultured using the cell culture substrate according to the present embodiment are not particularly limited as long as they can adhere to the surface before stimulation is applied by temperature drop. For example, in addition to various established cells such as Chinese hamster ovary-derived CHO cells, mouse connective tissue L929, human embryonic kidney-derived HEK293 cells, and human cervical cancer-derived HeLa cells, examples of the cells include epithelial cells and endothelial cells constituting each tissue and organ in a living body, skeletal muscle cells exhibiting contractility, smooth muscle cells, cardiomyocytes, neuron cells constituting the nervous system, glial cells, fibroblast cells, liver parenchymal cells involved in metabolism of a living body, liver non-parenchymal cells and adipocytes, as cells having differentiation potential, stem cells present in various tissues such as mesenchymal stem cells, bone marrow cells, and Muse cells, stem cells having pluripotency (pluripotent stem cells) such as ES cells and iPS cells, and cells induced to be differentiated therefrom.

The cell culture substrate according to the present embodiment can be produced, for example, by a production method including a covering step of covering at least a part of a surface of a substrate with a composition containing a hydrophilic polymer to form a layer containing a hydrophilic polymer, and a patterning step of subjecting a part of the surface of the layer containing a hydrophilic polymer to plasma treatment and forming an (A) region in a portion subjected to the plasma treatment.

In the covering step, at least a part of the surface of the substrate is covered with a composition containing a hydrophilic polymer to form a layer containing a hydrophilic polymer. By forming a layer containing a hydrophilic polymer, a state without cell adhesiveness and cell proliferative properties can be obtained. The method for forming the layer containing a hydrophilic polymer is not particularly limited, and examples thereof include a method in which the layer is formed by applying a composition containing a hydrophilic polymer to at least a part of the surface of the substrate. As a method for applying the composition containing the hydrophilic polymer, for example, various commonly known methods such as coating, brushing, dip coating, spin coating, bar coding, flow coating, spray coating, roll coating, air knife coating, blade coating, gravure coating, micro gravure coating, and slot die coating can be used.

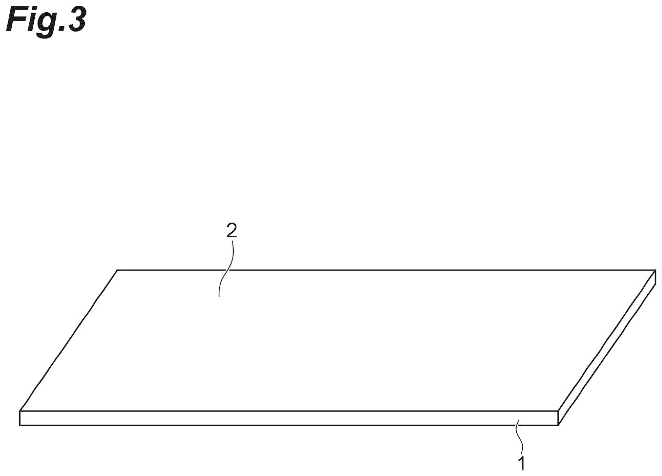

FIG. 3 is a schematic view (perspective view) of a substrate in which a layer containing a hydrophilic polymer is formed on a surface after a covering step. In FIG. 3, layer 2 containing a hydrophilic polymer is formed on the entire one surface of substrate 1.

When the hydrophilic polymer has reactivity to active energy rays, the covering step may include irradiating the layer containing the hydrophilic polymer with active energy rays to immobilize the layer containing the hydrophilic polymer on the surface of the substrate. Examples of the hydrophilic polymer having reactivity to active energy rays include hydrophilic polymers having a functional group having reactivity to the above-described active energy rays (for example, UV, electron beam, and the like). Examples of the active energy ray include UV and electron beam.

When the hydrophilic polymer having reactivity to the active energy ray is irradiated with the active energy ray, a chemical reaction between the hydrophilic polymers or between the hydrophilic polymer and the substrate occurs, and the layer containing the hydrophilic polymer is immobilized on the surface of the substrate. When the layer containing the hydrophilic polymer is immobilized on the surface of the substrate, the layer containing the temperature-responsive polymer can be formed without deforming the layer containing the hydrophilic polymer when the composition containing the temperature-responsive polymer is applied in the temperature-responsive layer forming step described later. In addition, the shape of the (A) region formed in the patterning step described later can be maintained by immobilizing the layer containing the hydrophilic polymer on the surface of the substrate.

The patterning step is a step of performing a plasma treatment on a part of the surface of the layer containing the hydrophilic polymer to form the (A) region in a portion subjected to the plasma treatment. The plasma irradiation time is preferably 5 seconds or more and 10 minutes or less, more preferably 10 seconds or more and 5 minutes or less, and most preferably 30 seconds or more and 3 minutes or less. As the introduction gas during the plasma treatment, air, nitrogen, oxygen, or water vapor is preferably used, and oxygen or water vapor is more preferably used. The gas pressure of the introduction gas is preferably 1 Pascal (Pa) or more and less than 20 Pascal, more preferably 1 Pascal or more and 15 Pascal or less, still more preferably 1 Pascal or more and 10 Pascal or less. By not excessively increasing the gas pressure, it is easier to set the inclination angle of the inclined surface in the (A) region within the above-described preferable range. Furthermore, since it becomes easier to set the inclination angle of the inclined surface in the (A) region within the above-described preferable range, the plasma treatment is particularly preferably performed by reactive ion etching treatment (RIE). RIE is also referred to as anisotropic etching. In addition, a method of patterning the (A) region is not particularly limited, and examples thereof include a method of performing plasma treatment on a desired portion (unmasked portion) by performing plasma treatment in a state of being covered with a metal mask, a silicon mask, a surface protective film, or the like.

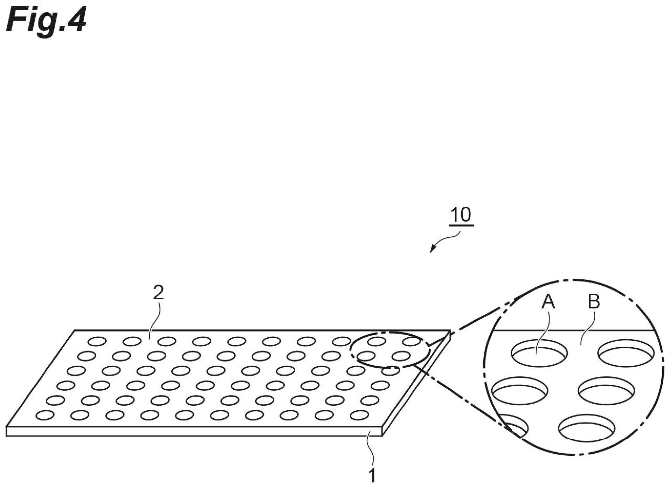

FIG. 4 is a schematic view (perspective view) of the cell culture substrate after the patterning step. In the cell culture substrate 10 shown in FIG. 4, regions in a circular shape arranged at equal intervals (regions indicated by A. For example, the area of the region is 0.001 mm² or more and 5 mm² or less) corresponds to the portion subjected to the plasma treatment. The region indicated by A comes to have cell adhesiveness and cell proliferative properties due to the modification of the surface of the layer 2 containing the hydrophilic polymer by plasma treatment. In addition, a region other than the region in a circular shape (Region indicated by B.) corresponds to a portion that is not subjected to plasma treatment by being protected by, for example, a metal mask or the like. The region indicated by B is the layer 2 containing a hydrophilic polymer on the surface, and thus does not have cell adhesiveness or cell proliferative properties.

After the patterning step, a step of washing the hydrophilic polymer with a solvent, dissolving the hydrophilic polymer not immobilized on the surface, and removing the hydrophilic polymer from the surface of the substrate may be performed. By removing the unimmobilized hydrophilic polymer, the surface of the substrate can be exposed to form the (A) region. As the solvent to be used, water and an alcohol are preferably contained since the solvent is suitable for removing a compound by-produced by self-reaction of a hydrophilic polymer having reactivity to active energy rays, for example, a compound containing nitrene derived from an azide group. By washing using a mixed solvent of water and alcohol, the hydrophilic polymer-derived component remaining in the (A) region can be reduced, and the cell adhesiveness and cell proliferative properties of the (A) region can be enhanced. As the alcohol, a lower alcohol is preferable, and for example, methanol, ethanol, 2-propanol, t-butanol, isobutanol, pentanol, and hexanol are preferable, and methanol or ethanol is more preferable. The content of the alcohol is preferably 50 to 95%, more preferably 50 to 90%, particularly preferably 60 to 90%, and most preferably 70 to 90%.

The production method according to another embodiment may form the shape of the (A) region only by the covering step without including the patterning step. In this case, as a covering method in the covering step, for example, injection printing such as inkjet printing or on-demand printing, letterpress printing such as flexographic printing or letterpress printing, intaglio printing such as gravure printing or pad printing, flat plate printing such as offset printing, or stencil printing such as screen printing can be used. Making the (A) region a region having cell adhesiveness and cell proliferative properties by surface modification may be performed before the covering step or after the covering step. The surface modification can be performed by, for example, corona discharge treatment, plasma irradiation, ultraviolet irradiation, or the like.

The production method according to the present embodiment may further include a bonding step and a temperature-responsive layer forming step as necessary in addition to the covering step and the patterning step.

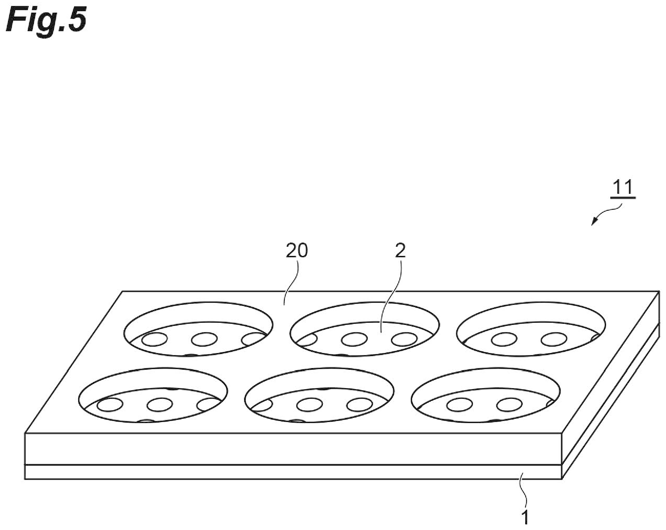

The bonding step is a step of bonding, after the patterning step, a plate having a through-hole having a cross-sectional area in the in-plane direction of 0.05 cm² or more and 100 cm² or less to the substrate on a surface side of the substrate covered with a layer containing a hydrophilic polymer. By bonding a plate having a through-hole having a cross-sectional area in an in-plane direction of 0.05 cm² or more and 100 cm² or less to a substrate, a plate having a space for containing a culture medium can be produced with high mass productivity. FIG. 5 is a schematic view (perspective view) of the cell culture substrate after the bonding step. The cell culture substrate 11 illustrated in FIG. 5 is obtained by, for example, bonding a partition plate 20 (a plate having a through-hole having a cross-sectional area in an in-plane direction of 0.05 cm² or more and 100 cm² or less) to the cell culture substrate 10 or the like illustrated in FIG. 4. The plate having a through-hole having a cross-sectional area in the in-plane direction of 0.05 cm² or more and 100 cm² or less may be, for example, a partition member having a plurality of cylindrical partition walls capable of partitioning a surface on a side for culturing cells. The side wall of the through-hole having a cross-sectional area of 0.05 cm² or more and 100 cm² or less functions as a cylindrical partition wall.

The temperature-responsive layer forming step is a step of, after the patterning step, covering the surface of the layer containing the hydrophilic polymer subjected to the plasma treatment with a composition containing the temperature-responsive polymer to form a layer containing the temperature-responsive polymer. At this time, by forming the layer containing the temperature-responsive polymer to have a layer thickness of 100 nm or less, the surface of the formed (A) region is easily covered with the molecular chain of the temperature-responsive polymer in a sparse state, and it is suitable for imparting temperature-responsiveness while maintaining the function (having cell adhesiveness and cell proliferative properties) of the (A) region. In addition, by setting the layer thickness of the layer containing the temperature-responsive polymer to 1 nm or more, a cell culture substrate capable of imparting sufficient temperature-responsiveness and rapidly forming cell aggregates can be produced, which is suitable.

As a method for covering with a composition containing a temperature-responsive polymer, the same method as the method for applying a composition containing a hydrophilic polymer described above can be suitably used.

As a method for covering with a composition containing a temperature-responsive polymer, it is preferable to cover the entire surface of the cell culture substrate with a temperature-responsive substance. When the covering with the composition containing the temperature-responsive polymer is performed, it is possible to enhance the mass productivity of the cell culture substrate by performing covering with the composition using a commonly used coating method without performing patterning. In addition, by covering the entire surface of the cell culture substrate with the composition containing the temperature-responsive polymer, the temperature-responsiveness is imparted to the (A) region, and the (B) region is also covered with the temperature-responsive polymer. When the (B) region is covered with the temperature-responsive polymer, cell adhesiveness of the (B) region can be reduced.

The cell culture kit according to the present embodiment includes a cell culture substrate. The cell culture substrate may have a partition member having a plurality of cylindrical partition walls capable of partitioning a surface on a side for culturing cells provided on the cell culture substrate according to the present invention described above.

The cell culture kit according to the present embodiment may include a coating agent containing a temperature-responsive polymer or a temperature-responsive polymer in addition to the cell culture substrate. As a result, a researcher who performs culture can easily adjust the layer thickness of the temperature-responsive polymer according to the type of cell.

The coating agent may contain a solvent. Examples of the solvent that can be included in the coating agent include water, an organic solvent, and a mixed solution thereof. Examples of the organic solvent include alcohols such as methanol, ethanol, 1-propanol, 2-propanol, and 1-butanol; and acetonitrile, formamide, N,N-dimethylformamide, dimethylsulfoxide, tetrahydrofuran, 1,4-dioxane, and methyl ethyl ketone. A mixed solvent of water and an alcohol is preferably used since it is suitable for making the film thickness of the coating uniform. The content of the temperature-responsive polymer based on the total mass of the coating agent can be 0.1 to 50 wt%, 0.2 to 10 wt%, or 0.5 to 5 wt%.

The coating agent may contain other components other than the temperature-responsive polymer and the solvent. Examples of the other component include a component for enhancing cell adhesiveness, and examples thereof include a polymer composed only of a water-insoluble block segment.

### [Examples]

Hereinafter, the present invention will be described more specifically based on Examples. However, the present invention is not limited to the following Examples. Unless otherwise specified, commercially available reagents were used.

### [Example 1]

A 80wt% aqueous ethanol solution containing polyvinyl alcohol (BIOSURFINE(R)-AWP, manufactured by Toyo Gosei Co., Ltd.) having an azide group as a hydrophilic polymer at a solid content concentration of 0.6wt% was added dropwise in an amount of 0.9 mL to a polycarbonate film (trade name: Panlite (registered trademark), manufactured by Teijin Limited, thickness 0.18 mm) (substrate), and spin coated (2000rpm, 60 sec) with a spin coater (trade name: MS-B150, manufactured by Mikasa Co., Ltd.). The substrate was then allowed to stand for 1 hour under a high-pressure mercury lamp for UV irradiation to cure the hydrophilic polymer, thereby forming a hydrophilic polymer layer (layer thickness: 75 nm). A metal mask (spot center-to-center distance: 800 µm) having a plurality of circular holes (spots) each having a diameter of 0.2 mm was placed on the layer of the hydrophilic polymer layer, and plasma treatment was performed from above the metal mask using a plasma irradiation apparatus (manufactured by Samco Inc., trade name: Aqua Plasma (registered trademark) Cleaner AQ-500) to form a (A) region in the plasma-treated portion. In addition, a (B) region was formed in a portion masked with a metal mask. Conditions for the plasma treatment are shown in Table 1. The term "anisotropic (RIE)" in Table 1 refers to plasma treatment in which a radio-frequency voltage is applied to a device chamber in which a substrate is placed, thereby using an ion collision effect in addition to a chemical reaction by radicals. On the other hand, the term "isotropic" refers to plasma treatment using a chemical reaction by radicals in a state in which a radio-frequency voltage is not applied to the device chamber.

### [Examples 2 to 4]

A cell culture substrate was produced in the same manner as in Example 1 except that the conditions for the plasma treatment were changed as shown in Table 1.

### [Example 5]

A cell culture substrate was produced in the same manner as in Example 1 except that a plasma irradiation apparatus (manufactured by Samco Inc., trade name: 10-NR) was used in place of the plasma irradiation apparatus (manufactured by Samco Inc., trade name: Aqua Plasma (registered trademark) Cleaner AQ-500) and the conditions of the plasma treatment were as shown in Table 1.

### [Example 6]

A cell culture substrate was produced in the same manner as in Example 1 except that an ICP plasma irradiation apparatus was used, a metal mask (one having a plurality of circular holes (spots) with a diameter of 0.1 mm and a spot center-to-center distance of 500 µm) was used in place of a metal mask (one having a plurality of circular holes (spots) with a diameter of 0.2 mm and a spot center-to-center distance of 800 µm), and the plasma treatment conditions were as shown in Table 1.

### [Example 7]

The cell culture substrate of Example 6, which had been stored in an environment of 15-30°C and 40-90%RH for 6 months, was used.

### [Comparative Example 1]

A cell culture substrate was produced in the same manner as in Example 1 except that a plasma irradiation apparatus (manufactured by SAKIGAKE Semiconductor Co., Ltd., trade name: Plasma Etcher-CPE-400) was used in place of the plasma irradiation apparatus (manufactured by Samco Inc., trade name: Aqua Plasma (registered trademark) Cleaner AQ-500), a metal mask (one having a plurality of circular holes (spots) with a diameter of 0.1 mm and a spot center-to-center distance of 500 µm) was used in place of a metal mask (one having a plurality of circular holes (spots) with a diameter of 0.2 mm and a spot center-to-center distance of 800 µm), and the plasma treatment conditions were as shown in Table 1.

### [Comparative Examples 2 to 4]

A cell culture substrate was produced in the same manner as in Example 1 except that the hydrophilic polymer layer was not formed and the conditions of the plasma treatment were changed as shown in Table 1.

### [Comparative Example 5]

The polycarbonate film (no hydrophilic polymer layer, no plasma treatment) used in Examples 1 to 6 and Comparative Examples 1 to 4 was used.

### [Comparative Example 6]

The polycarbonate film having a hydrophilic polymer layer (layer thickness: 75 nm, without plasma treatment) used in Examples 1 to 6 and Comparative Examples 1 to 4 was used.

### <Evaluation of cell culture substrate>

### (Measurement of layer thickness of hydrophilic polymer layer)

The layer thickness of the hydrophilic polymer layer formed on the surface of the substrate was determined by calculating the covering amount per unit area with the specific gravity of the polymer as 1 from the surface area of the substrate, the concentration of the hydrophilic polymer in the solution, and the volume of the solution dropped on the substrate. The results are shown in Table 1.

### (Evaluation of shape of (A) region)

The shape of the (A) region formed by etching by plasma treatment was measured using a stylus profiling system (manufactured by Bruker Corporation, trade name: DEKTAK XT). The cell culture substrate was fixed on a glass substrate with a tape, and measurement was performed under the following conditions. Note that the scanning direction is a direction along an axis passing through the center of the bottom surface (center of gravity) of the (A) region.
Z-axis measurement range: 6.5 µm
X-axis measurement range: 500 µm
Measurement time: 30 seconds
Loading force: 1 mg

From the obtained profile, the maximum depth (nm), the etching inclination (µm-Z-axis/mm-X-axis), and the inclination angle (°) were obtained.

The maximum depth was defined as the perpendicular distance at the point where the perpendicular distance from the interface on the opposite side to the surface of the hydrophilic polymer layer in contact with the substrate was maximum. That is, the distance is synonymous with the distance in the out-of-plane direction between the bottom surface of the (A) region and the surface of the (B) region.

The etching inclination is an inclination of an approximate straight line obtained by linearly approximating (least squares method) the inclined surface of the (A) region in the range of the depth of 20% or more and 70% or less when the maximum depth is 100%.

The inclination angle is an angle formed by the bottom surface of the (A) region and an approximate straight line.

The results are shown in Table 2.

### (Evaluation of amount of surface functional group in (A) region)

The amount of the surface functional group in the (A) region formed by the plasma treatment was measured by X-ray photoelectron spectroscopy. The polycarbonate film was subjected to a whole surface plasma or RIE treatment under the conditions in Table 1, and gas phase chemical modification using trifluoroethanol, di-t-butylcarbodiimide, and pyridine was performed to obtain a measurement sample. Measurement was performed under the following conditions using PH15000 VersaProbe II (manufactured by ULVAC-PHI).
X-ray source: monochrome Al-Kα ray (output: 25 W)
Analysis area: 1000 × 300 µm
Energy resolution: Wide scan spectrum 117.40 eV
High resolution spectrum 93.90 eV
Charge correction: C1s main peak (284.8 eV)

The carbon ratio (R_{COOH}) of carboxyl groups to the total carbon in the (A) region was calculated from the following formula derived from the reaction formula of gas phase chemical modification. 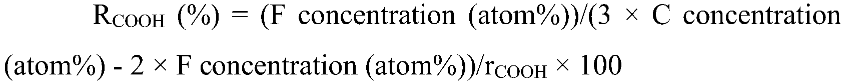 RCOOH (%) = (F concentration (atom%))/(3 × C concentration (atom%) - 2 × F concentration (atom%))/rCOOH × 100 r_{COOH}: Reaction rate (calculated from polyacrylic acid (standard ))

The results are shown in Table 3. In Comparative Examples 5 and 6, the region near the center of the film was measured.

### (Evaluation of water contact angle)

The water contact angle of the surface of the film surface-modified by the plasma treatment was evaluated using a contact angle measuring machine (model: DM300, manufactured by Kyowa Interface Science Co., Ltd.). A polycarbonate film was treated under the conditions in Table 1 to prepare a measurement sample, 1 µL of water was added dropwise to the surface-modified surface, and the water contact angle (°) after a lapse of 20 seconds was measured. The results are shown in Table 3. In Comparative Examples 5 and 6, the region near the center of the film was measured.

### (Evaluation of shape of opening)

A solution of a fluorescently labeled protein substrate for cell culture (manufactured by Matrixome, Inc., trade name: iMatrix-511) was added to the opening ((A) region) to adsorb the protein substrate. After removing the protein substrate solution and washing with PBS (-) (FUJIFILM Wako Pure Chemical Corporation), the cell culture substrate was observed from the hydrophilic polymer layer side in a direction orthogonal to the substrate using a fluorescence microscope, and a fluorescence image was captured. The major axis diameter (µm) and the minor axis diameter (µm) of the fluorescence image corresponding to each opening were measured, and the aspect ratio (major axis diameter (µm)/minor axis diameter (µm)) was calculated. Each cell culture substrate was measured at an n number of about 1200 to 1300, and an average value thereof was determined. In addition, CV (a value expressed as a percentage obtained by dividing the standard deviation by the average value) was also calculated. The results are shown in Table 2. Note that, since the protein substrate is adsorbed to the inclined surface and the bottom surface of the (A) region, a fluorescence image when the cell culture substrate is observed from the hydrophilic polymer layer side in a direction orthogonal to the substrate has a shape of an opening (a planar region defined by a boundary between the (A) region and the (B) region).

### (Evaluation of cell culture)

The cell culture substrate was attached to the bottom surface of a bottomless 6-well plate, and the sterilized product was used as a culture vessel. The human iPS cell 201B7 strain was seeded at 15000 cells/cm², and cultured in an environment of 37°C and a CO₂ concentration of 5% using an AK02N medium (manufactured by Ajinomoto Co., Inc.) (2 mL/well). Until 24 hours after the cells were seeded, Y-27632 (manufactured by Wako Pure Chemical Corporation) (concentration: 10µM) and a protein substrate for cell culture (manufactured by Matrixome Co., Ltd., trade name: iMatrix-511) (1.25 µg/mL) were added to the medium. After one day, three days, and five days from the start of culture, the culture medium was replaced. After 6 days from the start of the culture, the shape of the cell aggregate was evaluated by observation using a phase contrast microscope. In addition, the size of the cell aggregate (average of major axis diameter (µm) and minor axis diameter (µm)) was measured, and the enlargement rate (%) was calculated. The enlargement rate was defined as a value (percentage) obtained by dividing the size of the cell aggregate by the diameter of the spot of the metal mask. For each cell culture substrate, the size of the cell aggregate was measured and the enlargement rate was calculated at n = 15, and the average value thereof was obtained. The results are shown in Table 2.

**[Table 1]**

| | Plasma treatment conditions | | | | | | | Substrate conditions | |
|---|---|---|---|---|---|---|---|---|---|
| | Apparatus | Etching method | Output (W) | Gas type | Pressure (Pa) | Flow rate (ccm) | Treatment time (s) | Substrate thickness (µm) | Hydrophilic polymer layer thickness (nm) |
| Example 1 | AQ-500 | Anisotropic (RIE) | 250 | O₂ | 12 | 10 | 60 | 180 | 75 |
| Example 2 | AQ-500 | Anisotropic (RIE) | 250 | O₂ | 12 | 10 | 120 | 180 | 75 |
| Example 3 | AQ-500 | Anisotropic (RIE) | 250 | O₂ | 12 | 10 | 180 | 180 | 75 |
| Example 4 | AQ-500 | Isotropic | 250 | O₂ | 12 | 10 | 180 | 180 | 75 |
| Example 5 | 10-NR | Anisotropic (RIE) | 30 | O₂ | 30 | 2 | 360 | 180 | 75 |
| Example 6 | Dry etching apparatus | Anisotropic (RIE) | 4000 | O₂ | 1000 | 1.3 | 60 | 180 | 75 |
| Example 7 | The cell culture substrate 6 months after the treatment of Example 6 | | | | | | | | |
| Comparative Example 1 | CPE-400 | Anisotropic (RIE) | 400 | O2 | 20 | 30 | 60 | 180 | 75 |
| Comparative Example 2 | AQ-500 | Anisotropic (RIE) | 250 | O₂ | 12 | 10 | 60 | 180 | - |
| Comparative Example 3 | AQ-500 | Anisotropic (RIE) | 250 | O₂ | 12 | 10 | 120 | 180 | - |
| Comparative Example 4 | AQ-500 | Anisotropic (RIE) | 250 | O₂ | 12 | 10 | 180 | 180 | - |
| Comparative Example 5 | - | - | - | - | - | - | - | 180 | - |
| Comparative Example 6 | - | - | - | - | - | - | - | 180 | 75 |

**[Table 2]**

| | Shape evaluation of (A) region | | | Shape evaluation of opening | | | | Cell culture evaluation | |
|---|---|---|---|---|---|---|---|---|---|
| | Maximum depth (nm) | Etching slope (µm-Z-axis/mm-X-axis) | Inclination angle (°) | Diameter of opening of (A) region | Maximum depth | Aspect ratio | CV | Cell aggregate size (µm) | Enlargement rate (%) |
| Example 1 | 113.1 | 13.85 | 85.9 | 220.1 | 1946 | 1.04 | 4.09 | 267.2 | 133.6 |
| Example 2 | 255.5 | 43.86 | 88.7 | 217.7 | 852 | 1.04 | 5.59 | 267.4 | 133.7 |
| Example 3 | 342.0 | 14.04 | 85.9 | 241.4 | 705 | 1.03 | 2.66 | 269.1 | 134.6 |
| Example 4 | 104.0 | 2.3 | 66.5 | 294.0 | 2826 | 1.07 | 10.57 | 275.6 | 137.8 |
| Example 5 | 154.4 | 5.72 | 80.07 | 241.0 | 1560.6 | 1.05 | 6.90 | 252.7 | 126.4 |
| Example 6 | 130.5 | 7.52 | 82.19 | 137.3 | 1052.1 | 1.06 | 3.39 | 145.7 | 145.7 |
| Example 7 | Equivalent to Example 6 | | | | | | | 161.5 | 161.5 |
| Comparative Example 1 | 45.8 | 0.21 | 12.0 | 168.8 | 3685 | 1.35 | 23.67 | 185.1 | 185.1 |
| Comparative Example 2 | 113.1 | 13.85 | 85.9 | Evaluation impossible (Nonspecific adsorption) | | | | No aggregate formation | |
| Comparative Example 3 | 255.5 | 43.86 | 88.7 | Evaluation impossible (Nonspecific adsorption) | | | | No aggregate formation | |
| Comparative Example 4 | 342.0 | 14.04 | 85.9 | Evaluation impossible (Nonspecific adsorption) | | | | No aggregate formation | |
| Comparative Example 5 | No etching treatment (polycarbonate film) | | | | | | | No cell adhesion | |
| Comparative Example 6 | Hydrophilic polymer-covered film | | | | | | | No cell adhesion | |

**[Table 3]**

| | Analysis of amount of surface functional groups in (A) region | Hydrophilicity of (A) region | Cell culture evaluation | |
|---|---|---|---|---|
| | Carbon ratio of COOH groups to total carbon; R_{COOH} (%) | Water contact angle (°) | Cell aggregate size (µm) | Enlargement rate (%) |
| Example 1 | 1.2 | 64.4 | 267.2 | 133.6 |
| Example 2 | - | - | 267.4 | 133.7 |
| Example 3 | - | - | 269.1 | 134.6 |
| Example 4 | - | - | 275.6 | 137.8 |
| Example 5 | 0.80 | 28.4 | 252.7 | 126.4 |
| Example 6 | 0.50 | 44.80 | 145.7 | 145.7 |
| Example 7 | 0.30 | 65.20 | 161.5 | 161.5 |
| Comparative Example 1 | - | - | 185.1 | 185.1 |
| Comparative Example 2 | Equivalent to Example 1 | | No aggregate formation | |
| Comparative Example 3 | Equivalent to Example 2 | | No aggregate formation | |
| Comparative Example 4 | Equivalent to Example 3 | | No aggregate formation | |
| Comparative Example 5 | 0.1 | 87.7 | No cell adhesion | |
| Comparative Example 6 | 0.1 | 63.0 | No cell adhesion | |

In the cell culture substrates of Examples 1 to 3 and 5 to 7 in which the (A) region was formed by anisotropic etching, the inclination angle was large (85.9° to 88.7°), and a clear uneven shape was formed. In addition, in the cell culture substrate of Example 4 in which the (A) region was formed by etching while reducing the gas pressure although the etching was isotropic etching, the inclination angle was clearly larger and a clear uneven shape was formed as compared with the cell culture substrate of Comparative Example 1 in which the gas pressure was high. In addition, it can be understood that in the cell culture substrates of Examples 1 to 7, the aspect ratio of the shape of the opening was 1.1 or less; in the cell culture substrates of Examples 1 to 5, the enlargement rate of the cell aggregates was also 140% or less; and in the cell culture substrates of Examples 6 to 7, the enlargement rate of the cell aggregates was also 165% or less, and the (A) region was formed with a size and a shape close to the targeted size and shape (circular holes having diameters of 0.2 mm and 0.1 mm of the used metal mask). In particular, in the cell culture substrates of Examples 1 to 3 and 5 to 7, the CV of the aspect ratio of the shape of the opening was also small, and the size and shape were more uniform. In addition, regarding the ratio of the diameter/maximum depth of the opening of the (A) region, the substrate treated by the anisotropic etching and the isotropic etching under the low gas pressure condition had a value in the range of 500 or more and 3000 or less, and showed a clear uneven shape. On the other hand, in Comparative Example 1, it was confirmed that the etching inclination angle was small and the shape was unclear. In addition, since Comparative Examples 2 to 4 did not have the (B) region, cells adhered and proliferated nonspecifically outside the (A) region, and it was difficult to control the size. Furthermore, in Comparative Examples 5 to 6, since the substrates did not have the (A) region, the cells did not adhere and could not be cultured. The substrate surface etched under the treatment conditions of Examples 1 and 5 to 7 had a carbon ratio of carboxyl groups to total carbon (R_{COOH}) of 0.25% or more, and culture performance was good. In addition, as is apparent from the cell culture results, it is possible to more stably obtain cell aggregates having uniform size and shape by using these cell culture substrates.

### Reference Signs List

- A: (A) region
- A1: Inclined surface
- A2: Bottom surface
- B: (B) region
- H: Maximum depth of (A) region
- 1: Substrate
- 2: Layer containing hydrophilic polymer
- 10, 11: Cell culture substrate
- 20: Partition plate

## Claims

1. A cell culture substrate comprising:
a substrate, and a layer containing a hydrophilic polymer covering at least a part of a surface of the substrate, wherein the cell culture substrate has the following (A) region and (B) region:
(A) region having cell adhesiveness and cell proliferative properties
(B) region being adjacent to the (A) region and not having cell adhesiveness or cell proliferative properties; and
the (A) region includes a recess formed in the layer containing the hydrophilic polymer, the recess has an inclined surface and a bottom surface, and an inclination angle of the inclined surface is 40° or more and 110° or less.

2. The cell culture substrate according to claim 1, wherein a maximum depth of the (A) region is 1 nm or more and 500 nm or less.

3. The cell culture substrate according to claim 1 or 2, wherein at least a part of the bottom surface of the (A) region is composed of the substrate.

4. The cell culture substrate according to claim 1 or 2, wherein a layer thickness of the layer containing a hydrophilic polymer is 10 nm or more and 500 nm or less.

5. The cell culture substrate according to claim 1 or 2, wherein a planar region defined by a boundary between the (A) region and the (B) region is an ellipse having an area of 0.001 mm² or more and 5 mm² or less and an aspect ratio of 1 or more and 1.1 or less.

6. The cell culture substrate according to claim 5, wherein a ratio (diameter/maximum depth) of a diameter (nm) of an opening of the (A) region to a maximum depth (nm) of the (A) region is 500 or more and 3000 or less.

7. The cell culture substrate according to claim 1 or 2, wherein a minimum distance between the (A) regions is 400 µm or more and 10000 µm or less.

8. The cell culture substrate according to claim 1 or 2, wherein the water contact angle of the (A) region is 20° or more and 110° or less.

9. The cell culture substrate according to claim 1 or 2, wherein the (A) region is formed by reactive ion etching treatment.

10. The cell culture substrate according to claim 1 or 2, wherein a carbon ratio of carboxyl groups to total carbon in the (A) region (R_{COOH}) is 0.25% or more.

11. The cell culture substrate according to claim 1 or 2, wherein a thickness of the substrate is 0.01 mm or more and 0.5 mm or less.

12. A method for producing a cell culture substrate according to claim 1, comprising:
a covering step of covering at least a part of a surface of a substrate with a composition containing a hydrophilic polymer to form a layer containing the hydrophilic polymer; and
a patterning step of subjecting a part of the surface of the layer containing the hydrophilic polymer to plasma treatment to form the (A) region at the plasma-treated part.

13. The method for producing a cell culture substrate according to claim 12, wherein
the hydrophilic polymer has reactivity to an active energy ray, and
the covering step includes irradiating a layer containing the hydrophilic polymer with an active energy ray to immobilize the layer on a surface of the substrate.

14. The method for producing a cell culture substrate according to claim 12 or 13, wherein the plasma treatment is reactive ion etching treatment.

15. The production method according to claim 12 or 13, further comprising, after the patterning step, a bonding step of bonding a plate having a through-hole having a cross-sectional area in an in-plane direction of 0.05 cm² or more and 100 cm² or less to the substrate on a surface side of the substrate covered with the layer containing a hydrophilic polymer.

16. The production method according to claim 12 or 13, further comprising, after the patterning step, a temperature-responsive layer forming step of forming a layer containing a temperature-responsive polymer by covering a surface of the layer containing a hydrophilic polymer subjected to a plasma treatment with a composition containing the temperature-responsive polymer.

17. A cell culture kit comprising a cell culture substrate, wherein
the cell culture substrate has a partition member having a plurality of cylindrical partition walls capable of partitioning a surface on a side for culturing cells provided on the cell culture substrate according to claim 1 or 2.
